# EUROPEAN PATENT APPLICATION

(11) **EP 1 985 311 A1**
(43) Date of publication of application: **29.10.2008**
(21) Application number: 07707746.9
(22) Date of filing: 24.01.2007
(51) Int. Cl.: A61K 47/48, A61K 47/42, A61P 25/16, A61P 25/24, A61P 25/28, A61P 43/00

(54) **AGENT FOR TARGETING DRUG TO CEREBRAL NEURON**

(30) Priority: 24.01.2006 JP 2006015320
(71) Applicant: Kagoshima University, Kagoshima-shi, Kagoshima 890-8580 (JP)
(72) Inventor: KUCHIIWA, Satoshi, Kagoshima 890-8580 (JP); KUCHIIWA, Toshiko, Kagoshima 890-0075 (JP)
(74) Representative: Woods, Geoffrey Corlett
(86) International application number: PCT/JP2007/051528
(87) International publication number: WO 2007/086587

(57) **Abstract**

The present invention relates to targeting agents of drugs, more specifically, to targeting agents that cause drugs to be incorporated into brain neurons. The present invention further relates to medicines comprising the targeting agents and drugs. The present invention also relates to a method for targeting drugs to brain neurons.

## Description

### Technical Field

The present invention relates to targeting agents of drugs, more specifically, to targeting agents that cause drugs to be incorporated into brain neurons. The present invention further relates to medicines comprising the targeting agents and drugs. The present invention also relates to a method for targeting drugs to brain neurons.

### Background Art

Mental function takes place through activity of the neural network of the brain, and the role of bioactive substances such as neurotransmitters are crucial for regulation of such activity. Various psychiatric symptoms develop when the regulation of neurotransmitters or other bioactive substances becomes abnormal. Its examples include Parkinson's disease which is caused by deficiency of dopamine, Alzheimer's disease which is related to acetylcholine, and depression which is related to serotonin. A number of brain diseases have now been shown to be induced by dysregulation of neurotransmitters or other bioactive substances.

It may be theoretically assumed that many diseases caused by neurotransmitter abnormalities could be cured if the activities of neurotransmitters were restored to the normal condition. The brain, however, has a barrier called the "blood-brain barrier" that blocks certain substances (a mechanism where the neurons of the brain selectively incorporate only the required substances from the blood, and block the unnecessary or harmful substances). Therefore, drugs administered orally or through intravenous injection hardly reach a group of neuron populations that are the disease foci. For example, in the case of Parkinson's disease, which is caused by the lack of dopamine in the substantia nigra of the midbrain, it is known that the disease condition improves when the deficient dopamine is supplied to the substantia nigra, although medication with dopamine does not improve the symptoms. This is because many bioactive substances such as dopamine do not pass through the blood-brain barrier. Thus, oral administration and intravenous injection of drugs are almost ineffective in treating brain diseases.

Direct injection of the drug into the cerebrospinal fluid by lumbar puncture is one method of administering a drug to the brain. As the cerebrospinal fluid is present on the surface of both the brain and spinal cord, and the brain and spinal cord are secured in a state where they float in the cerebrospinal fluid, injection of a drug solution into the cerebrospinal fluid (by inserting a cannula into the lumbar cistern) can make the drug solution penetrate to the surface of the brain and its ventricles. However, a barrier called cerebrospinal fluid-brain barrier exists between the cerebrospinal fluid and the neurons, thus the drug cannot be delivered to specific sites in the brain satisfactorily.

Therefore, in order to treat brain diseases by medication, we have to overcome the problem of the blood-brain barrier or the cerebrospinal fluid-brain barrier.

As described above, in drug therapy for brain diseases, it is necessary to overcome the hurdle of the blood-brain barrier or the cerebrospinal fluid-brain barrier. Nevertheless, even if the drug passes straight through the blood-brain barrier or the cerebrospinal fluid-brain barrier, it is very likely that the drug would penetrate indiscriminately into all parts of the brain, causing serious adverse drug reactions. Therefore, it is preferable that the drug is incorporated into the neurons of the disease foci only and does not reach other normal neurons. For example, in patients with severe neurological symptoms because of deficient production of serotonin in the raphe nuclei, it is desirable to cause substances such as serotonin synthesis-related enzymes to be incorpoated into only the group of serotonin-producing neurons. In patients with severe dyskinesia caused by disturbances in the Purkinje cells of the cerebellum, it is preferable that the desired drug is delivered only to the Purkinje cells. Ideally, to minimize adverse drug reactions, and accurately control the dose, the administered drug should not be incorporated into other than the targeted neurons.

As described above, a cerebrospinal fluid-brain barrier exists on the surfaces of the brain and the ventricles, and the free flow of substances from the cerebrospinal fluid to the neurons is suppressed. Therefore, it has been believed until now that drugs administered into the cerebrospinal fluid could not be expected to be incorporated into the group of neuron population to be treated. However, as will be discussed later, research by the present inventors (which will be discussed later), and by other researchers, has demonstrated that specific substances are incorporated into certain neurons from the surface of the brain and the surfaces of ventricles, and accumulated in their cell bodies (Non-patent Document 1: Zhang et al., Brain Research, Vol. 989, p 1-8, 2003). For example, it has been reported that when glial cell line-derived neurotrophic factor (GDNF) is injected directly into the cerebrospinal fluid, GDNF is incorporated into brain parenchyma and the resultant uptake of GDNF by the brain parenchyma can improve the dopaminergic condition, a cause of Parkinson's disease (Non-patent Document 2: Lapchak et al., Brain Research, Vol. 747, 92-102, 1997). Moreover, it has been reported that when rhodamine-labeled microspheres, cholera toxin, and fluorogold were injected into the third ventricle, the label appeared in the raphe nuclei (Non-patent Document 3: Larsen et al., Neuroscience, Vol. 70, 963-988, 1996). However, there has been no report on methods of delivering drugs meant for treating brain neurons by using such substances.

### Disclosure of the Invention

Against the aforesaid background, the present invention aims at providing effective means for targeting a desired drug to neuron populations in the brain, and also providing medicines for effective prevention and treatment of diseases and disorders related to brain neurons.

As a result of painstaking investigations undertaken to solve the aforesaid problems, in which the cholera toxin B-subunit (CTB) was injected into the cerebrospinal fluid of test animals, and neuron populations examined for uptake of CTB, the present inventors confirmed that the CTB was incorporated into more than one group of brain neurons. Further, by injecting CTB or wheat germ agglutinin (WGA) coupled to enzymes into the cerebrospinal fluid of test animals, the present inventors confirmed uptake of the enzymes into their brain neurons. The present inventors thus discovered that targeting a desired substance (drug) to brain neurons was possible through the use of such substances that are incorporated into brain neurons. This led to the completion of the present invention.

In short, the present invention is agents for causing drugs to be incorporated into brain neurons, which are characterized in that they comprise substances that can be incorporated into brain neurons.

There is no particular restriction on the types of brain neurons targeted by the aforesaid targeting agents, and examples include cerebellar Purkinje cells, raphe nucleus neurons, cerebral cortex neurons, hypothalamus neurons, thalamus neurons, and brain stem neurons.

There is also no particular restriction on the substances that can be incorporated into brain neurons, and examples include enterotoxins and lectins. Here, it is preferable that the enterotoxin is detoxified. There is no particular restriction on the enterotoxins, and examples include cholera toxin (CT), heat-labile *Escherichia coli* enterotoxin (LT), heat-stable *Escherichia coli* enterotoxin (ST), *Staphylococcus aureus* enterotoxin (StE), and *Clostridium botulinum* enterotoxin. There are no particular restrictions on the lectins, and examples include *Lotus tetragonolobus* lectin, *Ulex europaeus* (common gorse) lectin, *Arachis hypogaea* (peanut) lectin, *Glycine max* (soy bean) lectin, *Ricinus communis* (castor bean) lectin, *Bauhinia purpurea* lectin, *Phaseolus vulgaris* (kidney bean) lectin, *Dolichos biflorus* (horse gram) lectin, *Sophora japonica* lectin, *Agaricus bisporus* lectin, *Canavalia ensiformis* (jack bean) lectin, *Lens culinaris* lectin, *Pisum sativum* (pea) lectin, *Vicia faba* (broad bean) lectin, *Triticum vulgare* (wheat germ) lectin, *Phytolacca americana* (pokeweed) lectin, *Solanum tuberosum* (potato) lectin, *Limulus polyphemus* lectin, and *Maackia amurensis* (amur maackia) lectin.

It is also possible to use the following substances as the substances that can be incorporated into brain neurons: fast blue, diamidino yellow, true blue, nuclear yellow, carbocyanines diL, diO and diA, fluorescent microspheres, PKH26, fluorogold, fluoro-emerald, microruby, microemerald, rhodamine B dextran, biocytin, primulin, evans blue, propidium iodide, bisbenzimide, albumin, tetanus toxin, horseradish peroxidase, and DAPI (4'-6-diamidino-2-phenylindole 2HCl).

Cholera toxin B-subunit (CTB) and wheat germ agglutinin (WGA) are suitable for use as the substances that can be incorporated into brain neurons, although such substances are not restricted to these.

The present invention is also medicines comprising the aforesaid targeting agents and drugs, used for targeted delivery of the drugs to neurons.

The targeting agents and the drugs are, for instance, directly coupled in the aforementioned medicines. Also, it is preferable to administer the medicines into the cerebrospinal fluid. This medicines can be one used for treatment or prevention of diseases or disorders arising, for instance, from serotonin dysregulation.

Furthermore, the present invention is a method for targeting drugs to brain neurons of a patient, which includes preparation of a medicine by coupling the targeting agent with a drug and administering the medicine into the cerebrospinal fluid of the patient.

The present invention provides agents for targeting drugs to brain neurons. These targeting agents can cause desired drugs to be incorporated only into specific brain neuron populations. Therefore, the present invention enables a drug to act specifically on the brain neurons, and reduce the effects of the drug on other cells.

### Brief Description of the Drawings

Figure 1A is an outline of the vehicle method of the present invention and the conventional drug delivery system.
Figure 1B is an illustration of the mechanism of vehicle (and drug) uptake by brain neurons.
Figure 2 shows photographs of an immunohistological samples that illustrate the uptake of the vehicle (CTB) by raphe nuclei (A) and cerebellar cortex Purkinje cells (B).
Figure 3 shows photographs of histochemical samples that illustrate the uptake of CTB-HRP by raphe nuclei (A) and cerebellar cortex Purkinje cells (B).
Figure 4 is a micrograph that shows the uptake of CTB-HRP by Purkinje cells.
Figure 5 shows photographs that illustrate uptake of WGA-HRP by raphe nuclei (A) and Purkinje cells (B).

### Best Mode for Carrying Out the Invention

The present invention is described in greater detail below. The present patent application claims the priority of Japanese Patent Application No. 2006-015320 filed on January 24, 2006, including the contents described in the description and/or drawings of the aforementioned patent application.

### 1. Outline of the vehicle method

The present invention provides new technology for preparing a medicine where a drug is coupled with a specific chemical substance (vehicle) and the drug is made to be incorporated into a specific group of neuron population in the brain, for therapy.

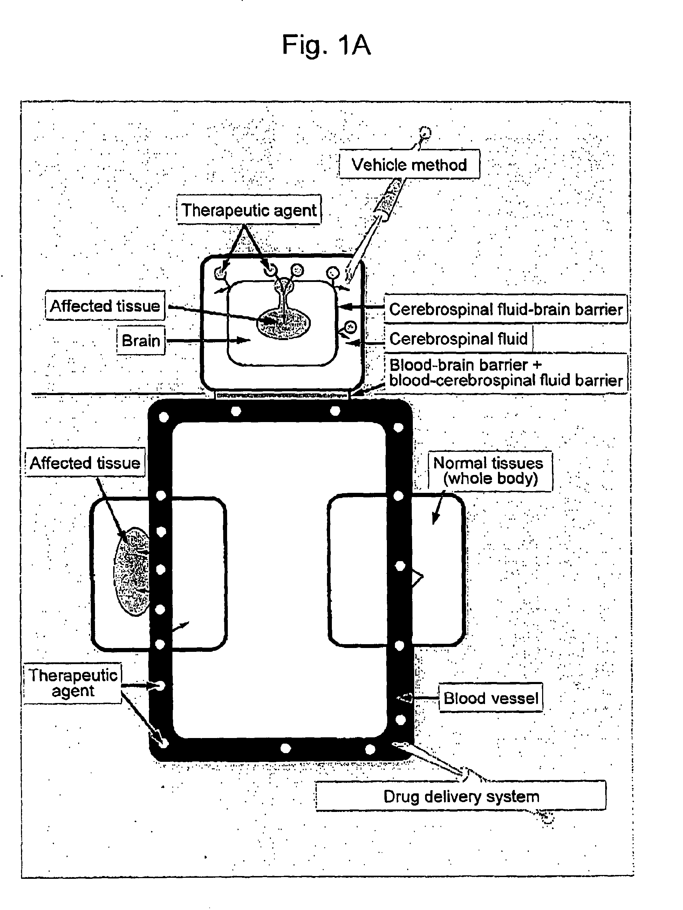

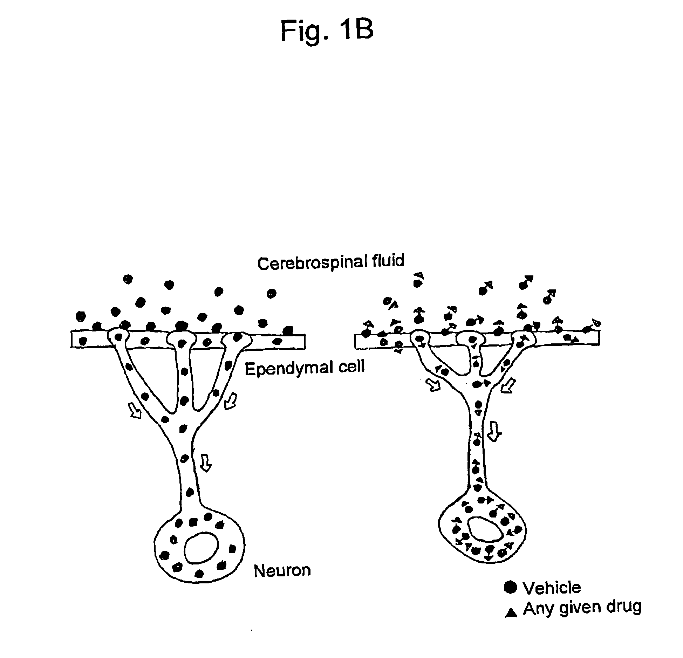

Earlier research by the present inventors had shown that certain neuron populations in the brain do not have the cerebrospinal fluid-brain barrier (these neuron populations are thought to have neurites projecting out on the brain surface or ventricle surface, through the walls of glial cells and ependymal cells). When certain substances are injected into the cerebrospinal fluid, they are incorporated into these neuron populations of the brain. Therefore, if a drug is administered coupled to such a specific substance into the cerebrospinal fluid by injecting, we can expect that the substance would be incorporated into the target group of brain neurons and the drug would be selectively delivered into the neurons (Figures 1A and 1B). In short, in spite of the presence of the cerebrospinal fluid-brain barrier, the drug will be incorporated into the brain and targeted to brain neuron populations.

Only brain neuron populations that do not have the cerebrospinal fluid-brain barrier can become targets of drug delivery, and this limits the range of application of the therapy. However, because the drug is not incorporated into other neurons, it has the advantage of considerably suppressing adverse drug reactions. Herein, the specific substance that is selectively incorporated into brain neurons, and thus plays the role of delivering the drug to the neurons is called a "vehicle", and a method of treatment using a medicine prepared by coupling a drug with a vehicle is called a "vehicle method".

The vehicle method is clearly different from the drug delivery system developed in the recent past, the practical use of which is advancing. In this conventional drug delivery system, after processing such as encapsulation of a small amount of a drug in small capsules (drug carriers) about several tens of nanometers across is performed, the drug is selectively delivered to the target cells such as those of cancerous tissue, or a drug is selectively delivered to target cells by exploiting interactive mechanisms such as the antigen-antibody reaction (see Figure 1A). Here, a mechanism of interaction, such as the antigen-antibody reaction, is utilized for selective delivery to target cells. In such a drug delivery system, it is a prerequisite to deliver a drug into a blood vessel. Therefore, a drug-loaded carrier needs to be of a size that is not recognized as a foreign body by the host and also of a size that is not filtered out by the kidneys.

Examples of carriers used in such a drug delivery system include (1) water-soluble polymers, (2) nano-sized particles (nanospheres), (3) small globules enclosed in lipid double-membranes (liposomes), and (4) polymeric micelles where polymers with heterogeneous structure are aggregated. This drug delivery system is a method in which the drug is coupled to the drug carrier and administered into a blood vessel to deliver it to the affected area via the circulatory system, and the drug is made to act on the targeted cells through chemical or physical mechanisms.

As far as selective administration of the drug to the targeted cells by coupling it to a macromolecular compound is concerned, the vehicle method of the present invention also has the same objective as the conventional drug delivery system. However, in the vehicle method, the drug is not administered into the circulatory system to be carried to the target cells, but is administered into the cerebrospinal fluid, and allowed to come into contact with the group of brain neurons that have neurites projecting into the cerebrospinal fluid, so that the drug is incorporated along with the vehicle into the brain neurons (Figure 1B). Unlike in the drug delivery system, in the vehicle method, the drug does not circulate in the entire body, and adverse drug reactions can be minimized. In the vehicle method of the present invention, there is no size restriction on the substance used as the vehicle, unlike with the drug carriers. Besides, there is no need for processing the drug, such as encapsulation. The hurdles of blood-brain barrier and cerebrospinal fluid-brain barrier have not been overcome in the currently used drug delivery systems. Therefore, the drug cannot be selectively administered to brain neurons. But this problem is solved in the vehicle method.

### 2. Vehicle

Vehicles that can be used in the vehicle method of the present invention are non-toxic substances (toxic substances can be used after removal of their toxicity), which can be incorporated into neurites of brain neurons, and get accumulated in the cells. Substances that reside in cells for a long time are preferable as vehicles. Some substances that can be used as vehicles are described below.

### (1) Enterotoxins

Among protein exotoxins produced by bacteria, those that cause diarrhea or vomiting by acting on the intestinal tract are collectively called enterotoxins. Examples of enterotoxins include substances produced by *Staphylococcus aureus* (StE), *Vibrio cholera* (CT), enterotoxigenic *Escherichia coli* (LT and ST), and *Clostridium botulinum.* These enterotoxins are substances that specifically bind to cell membranes (cell membrane-specific binding substances) and are incorporated efficiently into neurons. Therefore, it is very likely that such cell membrane-specific binding substances, like enterotoxins, are excellent vehicles. These enterotoxins can be used as vehicles after detoxification. For example, there have been reports of uptake, into brain neurons, of cholera toxin B-subunit (available from List Biochemical Laboratories; Coolen et al., J. Neurosci. Methods 91: 1-8, 1999) and heat-labile enterotoxin B-subunit (Okado et al., Neurosci. Lett. 120: 263-266, 1990). Regarding the enterotoxins derived from *Staphylococcus aureus* and *Clostridium botulinum,* there has been no report of their uptake into brain neurons. However, because they have an action mechanism in which they are incorporated into the gut walls and damage the nervous system, as with the cholera toxin and the heat-labile enterotoxin, it may be expected that these enterotoxins would also be incorporated into brain neurons when administered into the cerebrospinal fluid.

Both cholera toxin (CT) and the heat-labile enterotoxin (LT) consist of a toxic A-unit and a nontoxic B-unit that binds with cells. So, if the A-subunit is removed, the remaining B-unit is nontoxic and has the property of specifically binding with cell membranes. This method of detoxification of enterotoxins is well known in the art. The cholera toxin B-subunit, in particular, is a preferable vehicle because it is known to be an effective adjuvant in mucosal immunity and is used as a tracer in studies on neural circuits.

### (2) Lectins

Lectin is a collective term for sugar-binding proteins found in plants, animals, and bacteria. Lectins have the property of specifically binding to sugar chains present on the surface of cell membranes. Sugar chains occur on the surface of neurons as well, and a number of lectins are known to bind to neurons. It is highly likely that all of them can be used as vehicles. Examples of typical lectins of this type are listed below, along with information on where they can be obtained and examples of reports of their uptake by brain neurons, but they are not limited to these.
· L-fucose binding lectins: *Lotus tetragonolobus* lectin (Lotus), *Ulex europaeus* lectin (UEA-1; *Ulex europaeus* agglutinin, available from Vector Laboratories; Coulter et al., Anatomical Record 196: 36A-37A, 1980), etc;
· D-galactose, N-acetyl-D-galactosamine-binding lectins: *Arachis hypogea* (peanut) lectin (PNA; peanut agglutinin, available from Sigma; Coulter et al., Anatomical Record 196: 36A-37A, 1980), *Glycine max* (soybean) lectin (SBA; soybean agglutinin, available from Vector Laboratories; Borges and Sidman, J. Neurosci. 2: 647-653, 1982), *Ricinus communis* (castor bean) lectin (RCA120), *Bauhinia purpurea* lectin, *Phaseolus vulgaris* (kidney bean) lectin (*Phaseolus vulgaris* lectin with homoquadramer E-subunit (PHA-E₄), and *Phaseolus vulgaris* lectin (*Phaseolus vulgaris* lectin with homoquadramer L-subunit (PHA-L₄)), *Dolichos bifloris* (horse gram) lectin (DBA; *Dolichos bifloris* agglutinin, available from Vector Laboratories; Borges and Sidman, J. Neurosci. 2: 647-653, 1982), *Sophora japonica* lectin (SJA; *Sophora japonica* agglutinin, available from Vector Laboratories; Borges and Sidman, J. Neurosci. 2: 647-653, 1982), *Agaricus bisporus* lectin (ABA), etc;
· D-mannose-binding lectins: *Canavalia ensiformis* (jack bean) lectin (concanavalin A (Con A); available from Vector Laboratories; Phillipson and Griffiths, Brain Res. 265: 199-207, 1983), *Lens culinaris* lectin (LCA), *Pisum sativum* (pea) lectin, *Vieia faba* (broad bean) lectin, etc;
· Di-N-acetylchitobiose-binding lectins: *Triticum vulgare* (wheat germ) lectin (wheat germ agglutinin (WGA); available from Toyobo; Gonatas et al., J. Histchem. Cytochem, 27: 728-734, 1978), *Phytolacca americana* (pokeweed) lectin, *Solanum tuberosum* (potato) lectin, etc;
· Sialic acid-binding lectins: *Limulus polyphemus* lectin, *Maackia amurensis* (amur maackia) lectin (MAM), etc;

For example, wheat germ lectin, which is wheat germ agglutinin, is a protein of molecular weight about 36,000 that binds to the N-acetylglucosamine or N-acetylneuramic acid/ sialic acid components of glycoproteins or glycolipids on the surface of cell membranes. Wheat germ lectin bond to the cell membrane is incorporated into cell bodies, transported retrogradely through the neurites, and accumulated in the cell bodies. Therefore it is very likely that it would be an excellent vehicle.

### (3) Other substances

A substance can be used as a vehicle even if it does not specifically bind to the cell membrane, as long as it is incorporated into the neurites of brain neurons, and transported to be accumulated in the cells. However, because they do not specifically bind to cell membranes of brain neurons, it is believed that their uptake and amount transported would be relatively less. Examples of substances that are incorporated into and transported by neurites, and accumulated in the cell bodies of neurons include the following:

Fast blue (available from Sigma; Bentivvoglio et al., Neurosci. Lett. 18: 25-30, 1980), diamidino yellow (available from EMS-Polyloy; Puigdellivo-Sanchez et al., J. Neurosci. Methods 95: 103-110, 2000), true blue (available from Kirkegaard & Perry Laboratories and Sigma; Bentivvoglio et al., Neurosci. Lett. 18: 25-30, 1980), nuclear yellow (available from Hoechst; Bentivvoglio et al., Neurosci. Lett. 18: 25-30, 1980), carbocyanines, (diL, diO, diA, etc; available from Molecular Probes; Vidal-Sanz et al., Exp. Neurol. 102: 92-101, 1988), fluorescent microspheres (available from Tracer Technology; Katz et al., Nature 310: 498-500, 1984), PKH26 (available from Sigma; website http://www.med.kobe-u.ac.jp/anatol/Lib/Tract_tracing/index.html), fluoro-gold (available from Fluorochrome; Deng and Rogers, J. Neurosci. Methods 89: 75-86, 1999), dextrans (such as fluoro-emerald, microruby, microemerald, rhodamine B dextran, etc; available from Molecular Probes; Coolen et al., J. Neurosci. Methods 91: 1-8, 1999; Phillip and Powley, Autonomic Neuroscience: Basic and Clinical 123: 44-53, 2005, etc), biocytin (available from Molecular Probes; Izzo, J. Neurosci. Methods 36: 155-166, 1991), primulin (available from Aldrich Chemical; Kuypers et al., Neurosci. Lett. 6: 127-135, 1977), evans blue (available from Merck; Kuypers et al., Neurosci. Lett. 6: 127-135, 1977), propidium iodide (available from Sigma; Kypers et al., Neurosci. Lett. 12: 1-7, 1979), bisbenzimide (available from Hoechst; Bentivvoglio et al., Neurosci. Lett. 18: 19-24, 1980), albumin (available from Sigma; Kristensson, Acta Neuropathol. (Berl) 16: 293-300, 1970), tetanus toxin (available from Calbiochem; Schwab and Agid, Int. J. Neurol. 13: 117-126, 1979), horseradish peroxidase (available from Toyobo, Sigma, etc; Kristensson et al., Brain Res. 32: 399-406, 1971), and DAPI (4'-6-diamidino-2-phenylindole 2HCl) (Kuypers et al., Neurosci. Lett. 6: 127-135, 1977)

Many of these substances are used in the study of neural networks in brain science research, and they are known to be incorporated into neurites of brain neurons, and to accumulate in the cell bodies (see also Trojanowsky et al., J. Neurosci. Methods 9: 185-204, 1983).

### (4) Confirmation of function as a vehicle

The ability of the above-listed vehicles to be incorporated into brain neurons can be assessed using the technique known in the art. For example, the ability can be evaluated by injecting a substance to be tested into the cerebrospinal fluid of a test animal (mouse, rat, etc), as such or after labeling, and confirming whether or not the substance is uptake by brain neurons. Also, the uptake of the substance by brain neurons can be detected using antibodies against the test substance, or a label (fluorescent label, radioactive label, horseradish peroxidase label, alkaline phosphatase label, etc) attached to the test substance. Once it is confirmed that the substance is incorporated into brain neurons, the ability of the test substance as a vehicle can be evaluated by directly or indirectly binding a drug to the test substance, and confirming whether or not the drug is incorporated into the neurons. These procedures can be easily carried out by a person skilled in the art.

### (5) Targeting agent

The targeting agent of the present invention contains a vehicle that is incorporated into brain neurons as described above. Besides the vehicle, the targeting agent can contain linkers and the like used for binding with the drug. The present targeting agent is useful, because when administered after coupling the drug with the vehicle contained in it, it can target the drug to the brain neurons.

The brain neurons, which are the target of the drug, using the targeting agent, are not particularly limited, as long as the brain neurons have the property to selectively incorporate the vehicle. Examples of such brain neurons include cerebellar Purkinje cells, raphe nucleus neurons, cerebral cortex neurons (neocortex and paleocortex), hypothalamus neurons, thalamus neurons, and brain stem neurons, although not restricted to these.

### 3. Targeting a drug by using a vehicle

The medicine containing the vehicle and a drug can target the drug to brain neurons by exploiting the vehicle's property of being incorporated into the brain neurons. The drug may be coupled with the vehicle directly, or indirectly through a linker, etc.

A number of methods are available for coupling the drug to the vehicle, and any method known in the art may be used. For example, for direct coupling of the drug with the vehicle, the two can be linked through disulfide bonding or peptide bonding, etc, using amino groups, sulfone groups, carboxyl groups or the like present in the vehicle and the drug. Examples of effective and practical methods among such coupling methods are listed below, but they are not limited to these.
1) The method of oxidizing a sugar bound to the vehicle or the drug with periodic acid, to create an aldehyde group, and reducing the Schiff base formed through a reaction between the aldehyde group and an amino group of a protein of the other component to form a complex;
2) The method of using a pyridyl disulfide compound as a cross-linker. The link formed is a disulfide bond;
3) The method of preparing a complex by cross-linking through amino groups using p-benzoquinone;
4) The method in which a thiol group is created by reduction of a vehicle, which is then treated with an excess of N,N'-o-phenylenedimaleimide to introduce the maleimide group, and coupling it with the thiol group on the drug;
5) The method of coupling the vehicle and the drug through their amino groups and the SH-groups using a cross-linker. Compounds that can be used as cross-linkers (heterobifunctional reagents) for the amino and the SH groups include:
   N-(8-maleimidecapryloxy)sulfosuccinimide,
   N-(4-maleimidebutyryloxy)succinimide,
   N-(6-maleimidecaproyloxy)sulfosuccinimide,
   N-(4-maleimidebutyryloxy)sulfosuccinimide,
   N-(6-maleimidecaproyloxy)succinimide,
   N-(11-maleimideundecanoyloxy)succinimide,
   N-(8-maleimidecapryloxy)succinimide,
   N-(11-maleimideundecanoyloxy)sulfosuccinimide,
   N-succinimidyl-4-(N-maleimide)butyrate,
   N-succinimidyl-6-(N-maleimide)hexanoate, and
   N-sulfosuccinimidyl-4-(N-maleimidemethyl)cyclohexane-1-carboxylate; and
6) The glutaraldehyde method, in which the vehicle and the drug are treated with glutaraldehyde for coupling them.

When indirectly coupling the vehicle and drug, they may be coupled through a suitable linker, functional group, etc. There is no particular restriction on the linker or functional group, and any that does not affect the activity of the drug can be used. Such a linker or functional group can be easily selected by a person skilled in the art. For instance, the linker or functional group can be selected by first selecting the drug to be administered, selecting a vehicle compatible with the drug, coupling the vehicle and the drug through a linker or functional group, and administering it to an animal model to verify whether the drug activity is retained. Alternatively, it can be administered in the form of a prodrug where the drug is liberated from the vehicle after uptake by the brain neurons.

There is no particular restriction on the drug to be administered, as long as the drug is desired to be targeted to brain neurons. Besides, there is no particular restriction in the present invention about the size of the drug used. Examples of preferable drugs include:

### (1) Therapeutic agents for raphe nuclei diseases

Raphe nuclei are considered to be related to anxiety, depression, body temperature regulation, sleep and wake, regulation of circulatory organs, endocrine regulation, fear and stress, sexual behavior, mood disorders, and other mental activities. Many of these are considered to be related to regulation of serotonin produced by a group of raphe nucleus neurons. In diseases caused by dysregulation of the brain neurotransmitter serotonin (depression, panic disorder, obsessive compulsive disorder, generalized anxiety disorder, and serotonin syndrome, for instance), the symptoms are believed to improve in many cases with the administration of serotonin-related drugs (serotonin reuptake inhibitor, synthesis inhibitor, metabolic drugs, etc). So, it is expected that treatment would be possible by administering a serotonin-related drug coupled to a vehicle. Specific examples of such drugs include imipramine hydrochloride, amitriputyline hydrochloride, trimipramine maleate, fulvoxamine maleate, and paroxetine hydrochloride hydrate, though not limited to these. It is believed that there are a number of compounds other than these, which have not yet been used as drugs because they could not be targeted to brain neurons, but would be effective drugs for serotonin regulation if they could be targeted. Therefore, such compounds also can be used as drugs in the present invention.

### (2) Therapeutic agents for cerebellar Purkinje cell disorders

The cerebellum has the so-called the highest center of the extrapyramidal motor regulation in which the Purkinje cells play a central role. Malfunction in the cerebellum leads to intractable disease such as cerebellar degenerative disease, Machado-Joseph disease (MJD), and Friedreich ataxia, exhibiting a wide variety of symptoms like tremor, gait disturbance, dysstasia, shaking, vertigo, ocular motility disorder, autonomic nerve symptoms, peripheral nerve symptoms, extrapyramidal symptoms, and orthostatic hypotension. If drugs related to active substances concerned with the activity of cerebellar Purkinje cells can be administered after coupling with a vehicle, a new method of treating many cerebellar movement disorders would be opened up. Recently, it has been shown that mGluR1, a type of glutamate receptor, is essential for cerebellar Purkinje cells, and that it was possible to deliver substances related to synthesis or metabolism of this molecule to Purkinje cells selectively.

### (3) Therapeutic agents for other neuron populations

Research done by the present inventors showed that, besides the raphe nuclei and the cerebellar Purkinje cells, neuron populations in several other locations in the brain also incorporate vehicles. So, therapeutic agents delivered by the vehicle method can treat these other cell groups also. If the pathogenic mechanisms (neurochemical alterations or abnormalities of neuroactive substances such as neurotransmitters) that occur in such neuron populations get elucidated in the future, the development of therapeutic agents matching such mechanisms would become possible.

The medicine prepared as described above is administered to the cerebrospinal fluid. There is no particular restriction on the method of its administration, as long as it is a method of administering the medicine into the cerebrospinal fluid. For instance, it can be injected into a lumbar cistern by lumbar puncture (intrathecal administration). Lumbar puncture is a commonly used medical procedure that has little risk. Besides this, the medicine can be administered into the cerebrospinal fluid by ventricular puncture or ventricular catheter placement, from, for instance, an injection needle or a catheter inserted through the skull bone, or a reservoir placed beneath the skull bone. Moreover, a catheter can be inserted from the lumbar or sacral spine up to close to the ventricle to administer the medicine (transsacral or translumbar vertebra catheter method).

The medicine of the present invention can be any injectable dosage form (solution, emulsion, or suspension, for instance), and it may be provided as unit dose ampoules or in multiple dose containers. These formulations can contain solvents (distilled water, sterilized water, physiological saline, etc), surfactants, dispersants, buffers, pH-regulators, preservatives, solubilization assisting agents, absorption promoters, soothing agents, stabilizers, isotonic agents, etc, which are normally used in medicines. Such formulations can be prepared by a method known in the relevant technological field.

The amounts of vehicle and drug to be compounded in the medicine differ, depending on the type of brain neuron targeted, the disease to be treated, the body weight, age, and the state of disease of the patient to be administered, the method of administration, and the dose, etc. However, a person skilled in the art can determine the suitable amounts to be compounded, taking these factors into account.

Furthermore, the effective amount (dose) of the medicine and the time interval between administrations differ, depending on the type of vehicle and drug contained in the medicine, type of disease, the age, body weight, and state of disease of the patient, and the route and frequency of administration, and it can vary over a wide range.

This medicine is not particularly restricted regarding the type of subjects (patients) it can be used on. Examples include mammals, such as humans, livestock (cattle, swine, etc), pets (dogs and cats), and test animals (mice, rats, monkeys, etc). It is particularly preferable to use the medicine on patients affected by diseases or disorders related to brain neurons.

The medicine of the present invention can target the drug to brain neurons, and make it act specifically on the brain neurons. Therefore, it can reduce the effect of the drug on other neurons and thus avoid adverse drug reactions. Moreover, in the present invention, there is no restriction on the size of the drug to be targeted to the brain neurons, and any type of compound (such as proteins, antibodies, macromolecular compounds, etc) can be selected as the drug.

### Examples

The present invention is described below in detail by using some examples. However, the technical scope of the invention is not restricted by these examples.

### Example 1

In this example, the vehicle was injected into the cerebrospinal fluid to study the distribution of neuron populations that incorporate the vehicle. Thus, neuron populations that could become objects of targeting were identified.

### (1) Experimental procedure

A vehicle was injected into the cerebrospinal fluid of rats. After keeping the rats alive for a few days, histological samples of the brain were prepared to study the detailed distribution of cells that took up the vehicle. The vehicle used was cholera toxin B-subunit (CTB).

The specific experimental procedure used is described below. The rat was given general anesthesia with pentobarbital. It was placed in a stereotaxic head holder, an incision was made on skin of the head aseptically, and a hole of diameter about 2 mm was made on the skull with a dental drill, for inserting the injection needle. The hole was located at one of the following positions, relative to ear bar zero of a brain atlas: (AP +8 mm, LAT 3 mm), (AP +5 mm, LAT 3 mm), (AP +3 mm, LAT 1.5 mm), (AP +8 mm, LAT 1.5 mm) and (AP +5 mm, LAT 1.5 mm). A Hamilton microsyringe fitted on the stereotaxic head holder was vertically inserted through the hole in the skull bone, the tip of the needle was advanced to the subarachnoid space at the skull base, and a total of 3 µl or 5µl of 2.5% CTB aqueous solution was injected into the cerebrospinal fluid, taking at least 10 minutes. After the injection, the injection needle was withdrawn slowly and the incision on the skin of the head was sutured.

After the surgery, the rat was kept alive for 3 to 7 days and then euthanized by administering an excess of anesthetic. The rib cage of the rat was opened and a drip needle was inserted into the heart. Then, blood was washed off by passing physiological saline. After that, a fixing solution, which was a mixture of 4% paraformaldehyde and 0.2% picric acid, prepared with phosphate buffer, was perfused for fixing. After fixing, the brain was removed, and a 30% sucrose solution in phosphate buffer was allowed to infiltrate into the brain for 3 days.

After this infiltration, the brain was frozen in a freezing microtome and 40 µm thick serial frontal sections were prepared. The sections were treated with aqueous hydrogen peroxide and goat serum, and then treated with anticholera toxin antibody (List Biochemical Laboratories) in a refrigerator for 3 days to allow the antigen-antibody reaction to occur. The sections were then thoroughly washed with phosphate-buffered saline, and treated with secondary antibody (anti-rabbit goat IgG) for one day in a refrigerator. The sections were then washed well, peroxidase-labeled using rabbit PAP (peroxidase-antiperoxidase) and color developed with diaminobenzidine (DAB) to make the cholera toxin-immunopositive cells visible. The sections were then mounted on glass slides and Nissl stained with thionine. The samples thus prepared were observed under an optical microscope and the distribution of CTB immunopositive substances was examined in detail for each case of the experiment.

### (2) Results

In the immunohistochemical samples, CTB immunoactivity was observed in the pia mater and glial cells on the brain surface, and in ependymal cells on the ventricle surface. Non-specific infiltration on the brain surface or ventricle surface from any part of the brain was not observed. From this, it was concluded that CTB does not infiltrate indiscriminately through the brain surface or the ventricle surface.

The following two nuclei or sites showed very prominent uptake in the parenchyma away from surfaces of the brain and ventricles:
1. Raphe nuclei
2. Cerebellar Purkinje cell layer

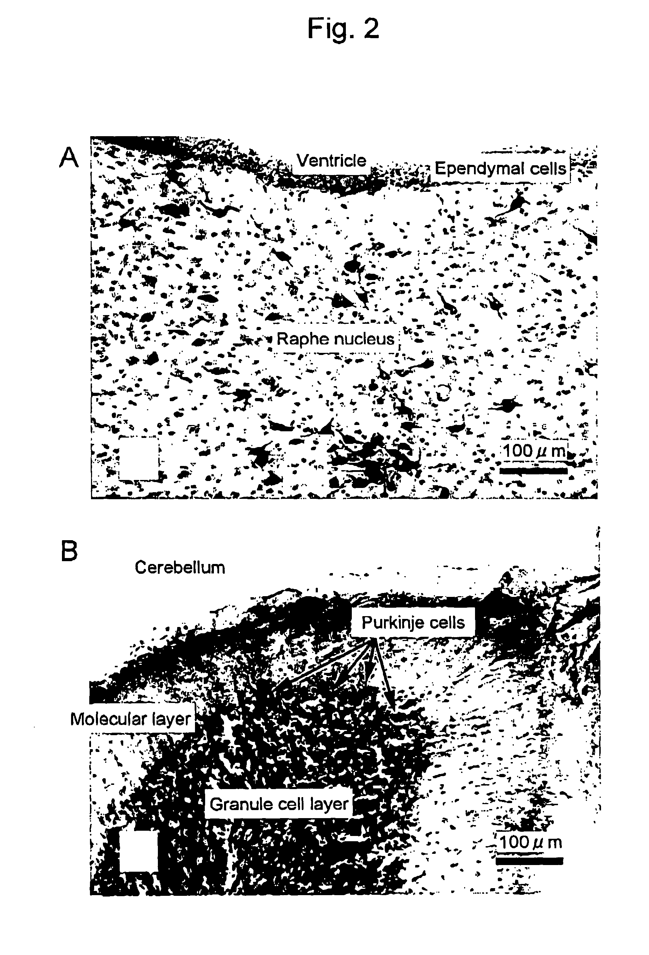

Very stable label was observed in these nuclei and the neuron populations in all the cases of the experiment (see Figures 2A and 2B). In the raphe nuclei, the projections extending from the labeled cells were observed to reach the surface of the ventricle (Figure 2A). As can be seen in Figure 2A, the neuron populations in the raphe nuclei are colored brown, which suggests that these cells had incorporate the vehicle. The cells stained blue had not incorporate the vehicle. This suggests that the vehicle had been incorporated into specific cells only, and that the vehicle did not penetrate indiscriminately into the brain parenchyma through the ependymal cells.

In the cerebellum, the label was seen in entire molecular layer and the Purkinje cells (Figure 2B). The brown particles, which mark the location of the vehicle, were seen particularly localized in the molecular layer and the Purkinje cell layer. The label in the cerebellum is believed to be the result of labeling of mainly the Purkinje cells and their dendrites. From the above observational findings, it was concluded that CTB had been incorporated from the surface of ventricles and the surface of the cerebellum, carried through the neurites, and accumulated in the neurons of the raphe nuclei and Purkinje cells.

The cell populations other than the aforesaid two neuron populations that were seen to have incorporated CTB were the following:
3. Cerebral neocortex
4. Cerebral paleocortex (septal nuclei, stria diagonalis, hippocampus, etc)
5. Hypothalamus (arcuate nuclei, tuber einereum, and mammillary bodies, etc)
6. Thalamus (anterior thalamic nuclei)
7. Brainstem (superior olivary nuclei, substantia nigra, ventral tegmental area, etc)

From the above results, it appeared that CTB did not unconditionally enter the brain from the brain surface or ventricle surface, and is incorporated only into cells that do not have the cerebrospinal fluid-brain barrier (see Figures 1A and 1B). Such neuron populations were distributed in a few other places in the brain, besides the raphe nuclei and the cerebellar Purkinje cell layer. This strongly suggested the possibility of using CTB as a vehicle for therapeutic agents for diseases in which these neuron populations were the disease foci. The fact that CTB activity could be demonstrated in the ventricle after it was injected into the subarachnoid space suggests that the test fluid containing the substance infiltrated into the ventricles against the flow of the cerebral fluid. This means that the vehicle is incorporated into the cells from the brain surface or the surface of a ventricle irrespective of the injection site, if it is injected into the cerebrospinal fluid.

### Example 2

The neuron populations that incorporate the vehicle were identified in Example 1. Therefore, in the current example, we confirmed that the drug coupled with the vehicle was in fact incorporated into those neuron populations.

### (1) Experimental procedure

Based on the results of Example 1, it may be expected that injecting the drug coupled with the vehicle into the cerebrospinal fluid would deliver the drug selectively to the target group of cells (i.e., cerebellar Purkinje cell layer, raphe nuclei, etc). To prove that this actually happens, a compound prepared by coupling CTB with horseradish peroxidase (HRP), i.e., CTB-HRP, was injected into the subarachnoid space of rats. HRP is a compound frequently used as a labeling substance in histological studies, and HRP in cells can be easily detected histochemically.

As in Example 1, the rat was anesthetized and surgery carried out after fixing the head in the stereotaxic head holder. The sites of injection were the same as in Example 1, but the dose of CTB-HRP (List Biochemical Laboratories) was 5 µl to 30 µl per rat. After the injection, the animal was kept alive for 3 to 5 days and then euthanized by administering an excess of anesthetic. The animal was then fixed by perfusing with phosphate buffer containing 1% paraformaldehyde and 1.25% glutaraldehyde, and the brain was taken out. After infiltrating the brain with 30% sucrose solution in phosphate buffer, 40 µm thick frozen serial frontal sections were prepared. The localization of cells containing CTB-HRP was then examined by the tetramethylbenzidine (TMB) method. The localization of CTB was examined immunohistochemically as in Example 1 and compared with the localization of HRP activity.

### (2) Results

In the histochemical samples of the TMB method, counterstaining with an ordinary dye is not performed. Therefore, the neurons cannot be seen under the microscope. The HRP-labeled granules can be seen in the samples. The presence of labeled cells can be confirmed by observing the distribution and localization of these granules.

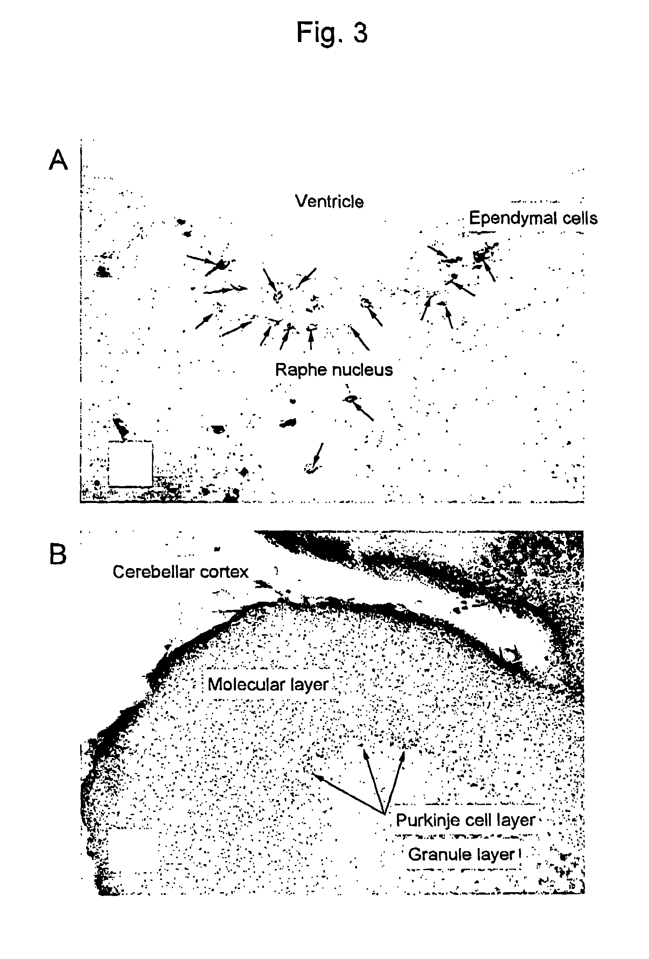

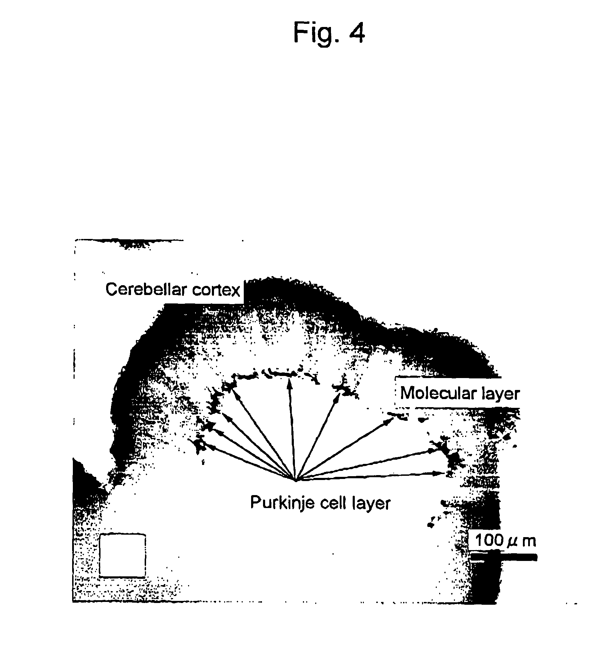

Figure 3A and 3B are photographs of histochemical samples of rats injected with CTB-HRP into the subarachnoid cerebrospinal fluid. Figure 3A shows the distribution of HRP-labeled granules in the region containing the dorsal raphe nucleus, located below a ventricle. A dense population of HRP-labeled granules can be seen matching with the shape of the neuron cell body and dendrites and clearly outlining the cell contours. From this image of the label distribution, it is clear that the group of cells of the dorsal raphe nucleus were specifically labeled. In other words, this result of the experiment proves that HRP coupled with CTB had been delivered without losing its activity, along with the CTB, into the neurons that constitute the dorsal raphe nucleus. Figure 3B is a micrograph of the cerebellar cortex from the same experiment. The HRP-labeled granules are distributed from the molecular layer to the Purkinje cell layer in the cerebellar cortex. The labeled granules are densely packed in the Purkinje cell layer, and the contours of the cell bodies are visible, although shown ambiguously. In order to compensate for the fuzziness of the HRP samples, CTB immunohistochemical assay was performed on adjacent sections. Figure 4 is a micrograph of one such adjacent section. CTB labeled granules were seen localized in the molecular layer to the Purkinje cell layer, and show more clearly the contours of the cells in the Purkinje cell layer. In other words, this suggests that HRP has been delivered to the Purkinje cell layer of the cerebellum also, without losing its activity.

This example proved that the HRP coupled with CTB was in fact delivered, without losing its activity, to the cerebellar Purkinje cell layer and the raphe nuclei. HRP alone also is incorporated into neurons, transported, and accumulated in cells. But a level of uptake
where the label could be detected was not confirmed with injection of minute amounts (this is the result of a comparative experiment carried out by the present inventors, the data are not given here). Therefore, the HRP label activity that was seen in the current experiment was due to HRP targeted by CTB. This experiment proved that CTB was a substance that could play the role of a vehicle. Therefore, medicines can be prepared by coupling various drugs, in place of the HRP, with CTB.

### Example 3

In this example, it was confirmed that other candidate compounds were also effective as vehicles for selective uptake of drugs.

### (1) Experimental procedure

Example 1 and Example 2 proved that cholera toxin B-subunit (CTB) was an effective vehicle. In this Example, it is experimentally proved that lectin compounds, which are other class of vehicle candidate compounds, were also effective for material transport. Wheat germ agglutinin (WGA) was used as the vehicle.

The rat was anesthetized as in Example 1, fixed in a stereotaxic head holder, and holes were made with a dental drill at the sites on the skull bone described in Example 1. Using a microsyringe fixed to the stereotaxic head holder, 2 mg of a substance prepared by coupling HRP with WGA (WGA-HRP, prepared by cross-linking, Toyobo) was dissolved in distilled water and injected into the subarachnoid space. After the injection, the incision on the skin over the skull was treated aseptically, and the animals were kept alive for 3 to 5 days. After completing this period, the animals were euthanized by administering an excess of anesthetic. Then blood was washed off by passing physiological saline from the heart, and the animals were fixed by perfusion of phosphate buffer of pH 7.4 containing 1% paraformaldehyde and 1.25% glutaraldehyde. After fixing, the brain was removed, and a 30% sucrose solution in phosphate buffer was allowed to infiltrate into the brain. Then, 40 µm thick serial frontal sections of the brain were prepared in a freezing microtome. The sections were treated with tetramethylbenzidine (TMB method) and examined for the distribution of HRP-positive granules under an optical microscope.

### (2) Results

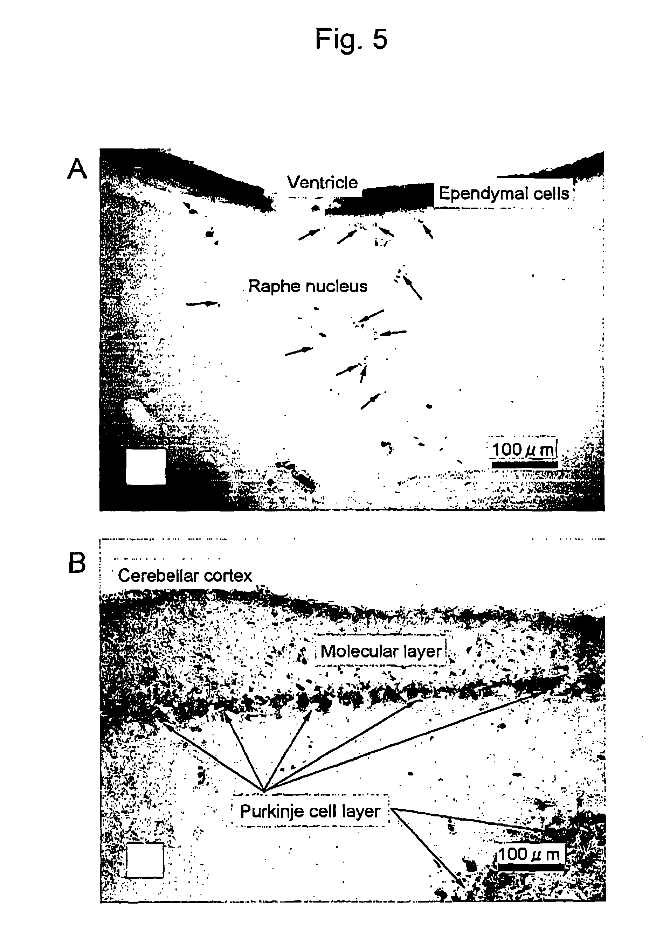

As in Example 2, no counterstaining was done in this experiment. So only the HRP-positive granules can be observed under the optical microscope. Populations of HRP positive granules were distributed particularly densely in the region from the molecular layer to the Purkinje cell layer in the cerebellar cortex and the raphe nucleus below the aqueduct of the midbrain (Figures 5A and 5B). This suggests that the HRP injected into the subarachnoid cerebrospinal fluid was incorporated, along with WGA, into the neuron populations of cerebellar Purkinje cells and raphe nuclei. In other words, the results prove that, like CTB, WGA is a substance that can effectively play the role of a vehicle for delivering drugs to target neurons.

All the publications, patents and patent applications cited herein will be incorporated entirely in the present specification.

### Industrial Applicability

The present invention provides an agent for targeting a drug to brain neurons. This targeting agent can cause a desired drug to be incorporated only into specific brain neuron populations. Therefore, the present invention enables the drug to act specifically on the brain neurons, and reduce the effects of the drug on other cells.

## Claims

1. A targeting agent for causing a drug to be incorporated into brain neurons, **characterized in that** it comprises a substance that can be incorporated into brain neurons.

2. The targeting agent according to claim 1, wherein the brain neurons are selected from the group consisting of cerebellar Purkinje cells, raphe nucleus neurons, cerebral cortex neurons, hypothalamus neurons, thalamus neurons, and brain stem neurons.

3. The targeting agent according to claim 1 or 2, wherein the substance that can be incorporated into the brain neurons is an enterotoxin or a lectin.

4. The targeting agent according to claim 3, wherein the enterotoxin has been detoxified.

5. The targeting agent according to claim 3 or 4, wherein the enterotoxin is selected from the group consisting of cholera toxin (CT), *Escherichia coli* heat-labile enterotoxin (LT), *Escherichia coli* heat-stable enterotoxin (ST), *Staphylococcus aureus* enterotoxin (StE), and *Clostridium botulinum* enterotoxin.

6. The targeting agent according to claim 3, wherein the lectin is selected from the group consisting of *Lotus tetragonolobus* lectin, *Ulex europaeus* (common gorse) lectin, *Arachis hypogaea* (peanut) lectin, *Glycine max* (soy bean) lectin, *Ricinus communis* (castor bean) lectin, *Bauhinia purpurea* lectin, *Phaseolus vulgaris* (kidney bean) lectin, *Dolichos biflorus* (horse gram) lectin, *Sophora japonica* lectin, *Agaricus bisporus* lectin, *Canavalia ensiformis* (jack bean) lectin, *Lens culinaris* lectin, *Pisum salivum* (pea) lectin, *Vicia faba* (broad bean) lectin, *Triticum vulgare* (wheat germ) lectin, *Phytolacca americana* (pokeweed) lectin, *Solanum tuberosum* (potato) lectin, *Limulus polyphemus* lectin, and *Maackia amurensis* (amur maackia) lectin.

7. The targeting agent according to claim 1 or 2, wherein the substance that can be incorporated into the brain neurons is selected from the group consisting of fast blue, diamidino yellow, true blue, nuclear yellow, carbocyanines diL, diO and diA, fluorescent microspheres, PKH26, fluoro-gold, fluoro-emerald, microruby, microemerald, rhodamine B dextran, biocytin, primulin, evans blue, propidium iodide, bisbenzimide, albumin, tetanus toxin, horseradish peroxidase, and DAPI (4'-6-diamidino-2-phenylindole 2HCl).

8. The targeting agent according to claim 1 or 2, wherein the substance that can be incorporated into brain neurons is cholera toxin B-subunit.

9. The targeting agent according to claim 1 or 2, wherein the substance that can be incorporated into brain neurons is wheat germ agglutinin.

10. A medicine comprising the targeting agent according to any one of claims 1 to 9 and a drug, for targeting the drug to brain neurons.

11. The medicine according to claim 10, wherein the targeting agent and the drug are directly coupled.

12. The medicine according to claim 10 or 11, which is administered into the cerebrospinal fluid.

13. The medicine according to any one of claims 10 to 12, which is used for treating or preventing a disease or disorder caused by serotonin dysregulation.

14. A method for targeting a drug to brain neurons of a patient, which comprises preparation of a medicine by coupling the drug with the targeting agent according to any one of claims 1 to 9, and administering the medicine into the cerebrospinal fluid of the patient.
